# EUROPÄISCHE PATENTSCHRIFT

(11) **EP 0 680 481 B1**
(45) Veröffentlichungstag und Bekanntmachung des Hinweises auf die Patenterteilung: **01.04.1998**
(21) Anmeldenummer: 94905039.7
(22) Anmeldetag: 12.01.1994
(51) Int. Cl.: C07D 491/04, C07D 221/08, A01N 43/90, A01N 43/42

(54) **DERIVATE DES AZAANTHRACHINONS UND DES AZAXANTHONS UND DIESE ENTHALTENDE HERBIZIDE UND PFLANZENWACHSTUMSREGULATOREN**
DERIVATES OF AZAANTHRAQUINONE AND AZAXANTHONE, HERBICIDES AND PLANT GROWTH REGULATORS CONTAINING THE SAME
DERIVES D'AZAANTHRAQUINONE ET D'AZAXANTHONE, HERBICIDES ET REGULATEURS DE CROISSANCE VEGETALE LES CONTENANT

(30) Priorität: 20.01.1993 DE 4301424
(43) Veröffentlichungstag der Anmeldung: 08.11.1995
(73) Patentinhaber: BASF AKTIENGESELLSCHAFT, 67056 Ludwigshafen (DE)
(72) Erfinder: PLATH, Peter, D-67227 Frankenthal (DE); RENTZEA, Costin, D-69123 Heidelberg (DE); MEYER, Norbert, D-68526 Ladenburg (DE); KAST, Juergen, D-67459 Boehl-Iggelheim (DE); KARDORFF, Uwe, D-68159 Mannheim (DE); GERBER, Matthias, D-67117 Limburgerhof (DE); WALTER, Helmut, D-67283 Obrigheim (DE); LANDES, Andreas, D-67117 Limburgerhof (DE); WESTPHALEN, Karl-Otto, D-67346 Speyer (DE)
(86) Internationale Anmeldenummer: EP9400073
(87) Internationale Veröffentlichungsnummer: WO9417070

(56) Entgegenhaltungen:
- WO-A-92/10500
- WO-A-92/22297
- DE-A- 2 210 654
- DE-A- 2 413 150
- COMPTES RENDUS HEBDOMADAIRES DES SEANCES DE L'ACADEMIE DES SCIENCES, SERIE C: SCIENCES CHIMIQUES Bd. 267 , Dezember 1968 , MONTREUIL FR Seiten 1826 - 1829 A. ETIENNE 'Sur quelques dérivés de la benzo(g)isoquinoléine'
- AUSTRALIAN JOURNAL OF CHEMISTRY Bd. 35, Nr. 7 , 1982 Seiten 1439 - 1450 D. W. CAMERON ET AL 'Nucleophilic alkenes. IX. Addition of 1,1-dimethoxyethene to azanaphthoquinones: Synthesis of bostrycoidin and 8-O-methylbostrycoidin'
- JOURNAL OF NATURAL PRODUCTS Bd. 52, Nr. 5 , September 1989 Seiten 987 - 995 T. KONOSHIMA ET AL 'Studies on inhibitors of skin tumor promotion, VI. Inhibitory effects of quinones on Epstein-Barr virus activation'
- CHEMICAL ABSTRACTS, vol. 99, no. 15, 10. Oktober 1983, Columbus, Ohio, US; abstract no. 122334b, TAKANORI OE ET AL 'Studies on the synthesis and antiallergic activity of 5H-benzopyrano(2,3-b)pyridine derinatives' Seite 641 ; & YAKUGAKU ZASSHI Bd. 103, Nr. 3 , 1983 Seiten 300 - 312
- CHEMICAL ABSTRACTS, vol. 84, no. 21, 1976, Columbus, Ohio, US; abstract no. 150612, MICHIO NAKANISHI ET AL 'Benzopyranopyridine derivatives' Seite 548 ; & JP,A,50 130 794 (YOSHITOMI PHARMACEUTICAL INDUSTRIES) 16. Oktober 1975
- JOURNAL OF MEDICINAL CHEMISTRY. Bd. 18, Nr. 1 , Januar 1975 , WASHINGTON US Seiten 1 - 8 F. J. VILLANI ET AL 'Benzopyranopyridine derivatives.1. Aminoalkyl derivatives of the azaxanthenes as bronchodilating agents'
- CHIMIE THéRAPEUTIQUE Bd. 8, Nr. 6 , November 1973 Seiten 652 - 654 P. VALENTI ET AL 'Structure-activity relationships in centrally stimulating xanthone derivatives. Part IX. Azaxanthone derivatives'

## Beschreibung

Die vorliegende Erfindung betrifft Azaanthrachinone und Azaxanthone der Formel I 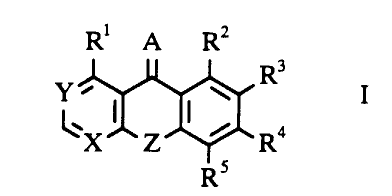 in der X, Y, Z und die Reste R¹ bis R⁵ folgende Bedeutung haben:
- R¹: Wasserstoff, Methyl,
- R²-R⁵: a) Wasserstoff,
b) Halogen,
c) Nitro,
d) CONH₂ und CONR⁶R⁷, wobei R⁶ und R⁷ C₁-C₄-Alkyl bedeuten,
e) C₁-C₈-Alkyl, das ein bis drei der folgenden Substituenten tragen kann: Halogen, Hydroxy, C₁-C₆-Alkoxy,
f) C₃-C₆-Alkenyl,
g) C₁-C₄-Alkoxy,
h) Hydroxy,
i) Amino oder NR⁶R⁷,
j) Phenyl, das ein bis fünf Halogenatome oder ein bis drei der folgenden Substituenten tragen kann: Nitro, C₁-C₄-Alkoxy, C₁-C₄-Alkyl oder C₁-C₄-Halogenalkyl;
- X, Y: N, N⁺-O⁻ oder CH, mit der Maßgabe, daß ausschließlich ein Stickstoffatom bzw. eine N-oxidgruppe im Ring enthaltend ist;
- Z: Sauerstoff oder Schwefel;
- A: Sauerstoff oder Schwefel;
sowie die Salze von I mit solchen Säuren, welche die herbizide bzw. die pflanzenwachstumsregulierende Wirkung von I nicht beeinträchtigen; mit der Maßgabe, daß das Alkaloid Cleistopholin sowie die Azaxanthone der Formel Ia 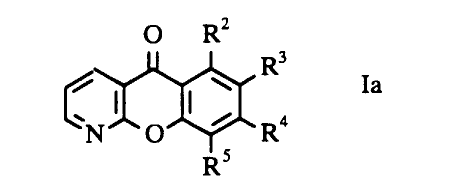 in der R², R³, R⁴, R⁵ gleich oder verschieden sind und unabhängig voneinander Wasserstoff, Fluor, Chlor, Methyl, Methoxy, CF₃, CCl₃, tert.-C₄H₉, NO₂, NH₂ und Phenyl bedeuten, vom Anspruch ausgeschlossen sind.

Aus den Arbeiten von P.G. Waterman et al (Phytochemistry, 24, 523 (1985)) und von A. Cavé et al (J. Nat. Prod. 50, 759 (1987)) ist das Alkaloid Cleistopholin oder 4-Methylbenzo[g]chinolin-5,10-dion bekannt, das aus der Pflanze Cleistopholis patens isoliert wurde. Bereits bekannt sind auch zahlreiche 5H-[1]Benzopyrano[2,3-b]pyridin-5-one aus den Arbeiten von F.J. Villani et al (J. Med. Chem., 18, 1 (1975)) und von P. Natka-Namirski (Acta Pol. Pharm. 34, 1 (1977)). Diesen Verbindungen werden in der genannten Literatur antihistaminische, bronchodilatatorische und anticandida Eigenschaften zugeschrieben. Eine herbizide bzw. pflanzenwachstumsregulierende Wirkung dieser Substanzen ist jedoch nicht bekannt.

Da die bekannten Verbindungen in ihrer Wirkung nicht immer befriedigen, lag der Erfindung die Aufgabe zugrunde, neue herbizide Mittel mit stärkerem herbiziden Effekt und neue Pflanzenwachstumsregulatoren, vorzugsweise Wachstumshemmer zur Verfügung zu stellen. Weiterhin lagen der Erfindung neue herbizid und pflanzenwachstumsregulierend wirkende Verbindungen als Aufgabe zugrunde.

Demgemäß wurden die eingangs definierten neuen Verbindungen I gefunden.

Weiterhin betrifft die Erfindung herbizide und bioregulatorisch wirkende, insbesondere des Wachstums der Pflanzen hemmende Mittel, enthaltend die Verbindungen I und Verfahren zur Herstellung der Verbindungen I. Darüber hinaus wurde gefunden, daß die Verbindungen der Formel I', die den Verbindungen der Formel I, einschließlich den Verbindungen Ia entsprechen, zur Verwendung als Herbizide bzw. Pflanzenwachstumsregulatoren geeignet sind. Eine entsprechende neue Wirkung zeigt auch das bekannte Alkaloid Cleistopholin.

Bevorzugte neue Verbindungen der Formel I sind solche, in denen die Substituenten folgende Bedeutung haben:
- R¹: Wasserstoff und insbesondere Methyl,
- R²,R³,R⁴,R⁵: Wasserstoff, Fluor, Chlor, Brom, unverzweigtes oder verzweigtes C₁-C₈-Alkyl, insbesondere C₁-C₆-Alkyl wie Methyl, Ethyl, n-Propyl, iso-Propyl, n-Butyl, sek.-Butyl, iso-Butyl, tert.-Butyl, n-Pentyl, iso-Pentyl, 1,1-Dimethylpropyl, 2,2-Dimethylpropyl, n-Hexyl, 1-Ethyl-1-methylpropyl;
eine unverzweigte oder verzweigte C₁-C₈-Alkylgruppe, insbesondere eine C₁-C₆-Alkylgruppe, die bis zu 3 der folgenden Substituenten tragen kann: Halogen wie Fluor, Chlor oder Brom, Hydroxyl, C₁-C₆-Alkoxy, insbesondere C₁-C₄-Alkoxy wie Methoxy, Ethoxy und Propoxy;
eine unverzweigte oder verzweigte C₃-C₆-Alkenyl-gruppe, insbesondere Vinyl, Allyl, 2-Methylallyl, 3-Methylallyl, 2,3-Dimethylallyl, 3,3-Dimethylallyl, 2-Pentenyl und 3-Pentenyl;
eine C₁-C₄-Alkoxygruppe, insbesondere Methoxy, Ethoxy und Propoxy;
Nitro, Amino (NH₂) ; Amide wie CO-NH₂ oder CO-N(CH₃)₂ und CO-N(C₂H₅)₂;
eine Phenylgruppe, ggf. ein bis dreimal durch Fluor, Chlor, CF₃, NO₂ oder C₁-C₄-Alkyl substituiert;
- R⁶, R⁷: eine unverzweigte oder verzweigte C₁-C₄-Alkylgruppe wie Methyl, Ethyl, n-Propyl, iso-Propyl, n-Butyl, iso-Butyl, sek.-Butyl und tert.-Butyl;
- X: N, N⁺-O⁻ oder CH;
- Y: CH, N oder N⁺-O⁻; und
- Z: Sauerstoff oder Schwefel
- A: Sauerstoff oder Schwefel.

Besonders bevorzugt sind Verbindungen der Formel I, in der R¹ Methyl bedeutet. Ferner steht X bevorzugt für Stickstoff oder die N-oxidgruppe und Y für CH.

Das Substitutionsmuster im Phenylring der Formel I weist insbesondere Mono- oder Disubstitution auf, wenn nicht alle Reste R² bis R⁵ Wasserstoff bedeuten. Als Substituenten kommen bevorzugt Halogenatome wie Fluor, Chlor oder Brom; C₁-C₄-Alkylreste, z.B. verzweigte Alkylreste wie Isopropyl, sec.-Butyl und tert.-Butyl, C₁-C₄-Halogenalkylreste, insbesondere Trifluormethyl und die Nitrogruppe in Betracht. Vorteilhaft sind auch Verbindungen in denen R² und R⁴ Wasserstoff bedeuten und R³ und/oder R⁵ einen der genannten Substituenten bedeutet.

Als Säureadditionssalze eignen sich die Salze von solchen Säuren, welche die herbizide Wirkung der Verbindungen I und Ia nicht beeinträchtigen, also z. B. die Hydrochloride und -bromide, Sulfate, Nitrate, Phosphate, Oxalate oder die Dodecylbenzolsulfonate.

Die Azaanthrachinone und Azaxanthone Ia sind bekannt oder (im Falle I) in an sich bekannter Weise erhältlich.

In den Fällen, in denen Y = CH, X = N, R¹ = CH₃ und Z die Gruppe C=O bedeutet, kann man die Verbindungen I und Ia nach folgendem Schema herstellen: 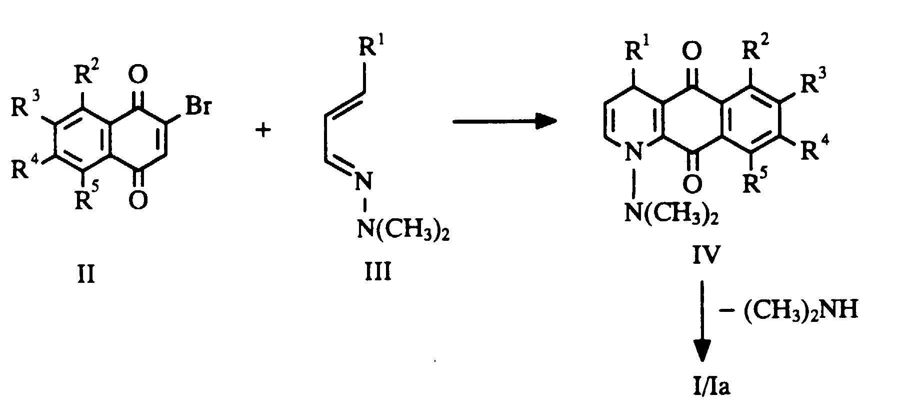 Dieses Verfahren ist z. B. aus den Arbeiten von F. Bracher, Liebigs Ann. Chem. 1989, 87 bekannt.

Die Hetero-Diels-Alder-Cycloaddition von 2-Brom-1,4-naphthochinonen (II) mit Crotonaldehyd-dimethylhydrazon (III) ergibt unter HBr-Abspaltung das Dihydropyridin (IV). Die thermische Eliminierung von Dimethylamin aus (IV) ergibt die Verbindungen I bzw. Ia. Diese Synthese wird durch mehrstündiges Erhitzen von (II) mit (III) auf Temperaturen von 100 bis 160°C in einem inerten organischen Lösungsmittel vorgenommen. Besonders vorteilhaft erfolgt die Cycloaddition und Eliminierung im Eintopfverfahren, d.h. ohne Isolierung des Zwischenproduktes IV. Als inerte organische Lösungsmittel bieten sich insbesondere Xylol, Toluol, Ethyl-benzol, Isopropylbenzol, Chlorbenzol oder Chlortoluol an.

In den Fällen, in denen Y = CH, X = N, R¹ = H oder CH3 und Z Sauerstoff bedeutet, werden die Azaxanthonen I und Ia bevorzugt nach dem Verfahren von F.J. Villani et al (loc. cit.) hergestellt:

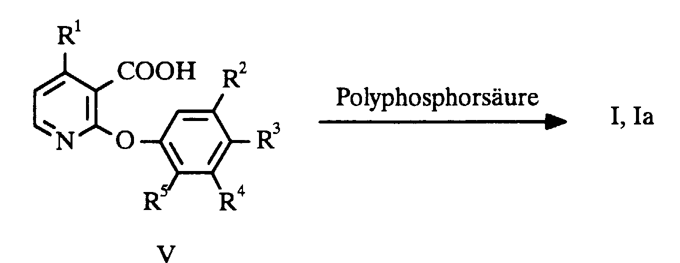

Die Cyclisierung der bekannten 2-Phenoxynicotinsäure (V) zu den Azaxanthonen I und Ia wird in Polyphosphorsäure bei Temperaturen von 100 bis 180°C durchgeführt, wobei die Polyphosphorsäure vorteilhaft auch als Lösungs- und Verdünnungsmittel verwendet werden kann.

Die Synthese der Verbindungen I mit X oder Y = N⁺-O⁻ erfolgt durch Umsetzung der entsprechenden Azaanthrachinone und Azaxanthone mit H₂O₂ oder mit organischen Peroxysäuren nach Standardverfahren (s. z. B. E. Ochiai, "Aromatic Amine Oxides", Elsevier, Amsterdam, 1967, S. 200-250).

Zur Herstellung von Mitteln zur Bekämpfung unerwünschten Pflanzenwuchses oder zur Regulierung des Pflanzenwuchses werden bevorzugt Verbindungen der Formel I verwendet, in der R¹ Wasserstoff, R² Wasserstoff oder Halogen, R³ Wasserstoff, Halogen, C₁-C₄-Alkyl oder Nitro, R⁴ Wasserstoff, Halogen, C₁-C₄-Alkyl oder -Alkoxy oder Hydroxy, R⁵ Wasserstoff oder Halogen, X N, Y CH und Z Sauerstoff, Schwefel oder C=O und A Sauerstoff oder Schwefel bedeuten.

Im Hinblick auf die biologische Verwendung bevorzugte Verbindungen I und I' sind in nachfolgender Tabelle 1 aufgeführt:

**Tabelle 1**

| R¹ | R² | R³ | R⁴ | R⁵ | X | Y | Z |
|---|---|---|---|---|---|---|---|
| CH₃ | H | H | H | H | N | CH | C=O |
| CH₃ | H | H | H | H | CH | N | C=O |
| CH₃ | H | H | H | H | N⁺-O⁻ | CH | C=O |
| H | H | H | H | H | N | CH | O |
| H | H | H | H | H | N⁺-O⁻ | CH | O |
| CH₃ | H | H | H | H | N | CH | O |
| CH₃ | H | H | H | H | N⁺-O⁻ | CH | O |
| CH₃ | H | H | H | H | CH | N | O |
| CH₃ | H | H | H | H | CH | N⁺-O⁻ | O |
| CH₃ | F | H | H | H | N | CH | C=O |
| H | F | H | H | H | N | CH | O |
| CH₃ | F | H | H | H | N | CH | O |
| CH₃ | F | H | H | H | CH | N | C=O |
| CH₃ | F | H | H | H | CH | N⁺-O⁻ | C=O |
| CH₃ | H | F | H | H | N | CH | C=O |
| H | H | F | H | H | N | CH | O |
| CH₃ | H | F | H | H | N | CH | O |
| CH₃ | H | F | H | H | CH | N | C=O |
| CH₃ | H | F | H | H | CH | N | O |
| CH₃ | H | H | F | H | N | CH | C=O |
| H | H | H | F | H | N | CH | O |
| CH₃ | H | H | F | H | N | CH | O |
| CH₃ | H | H | F | H | CH | N | C=O |
| CH₃ | H | H | H | F | N | CH | C=O |
| H | H | H | H | F | N | CH | O |
| CH₃ | H | H | H | F | N | CH | O |
| CH₃ | H | H | H | F | CH | N | C=O |
| CH₃ | Cl | H | H | H | N | CH | C=O |
| H | Cl | H | H | H | N | CH | O |
| CH₃ | Cl | H | H | H | N | CH | O |
| CH₃ | Cl | H | H | H | CH | N | C=O |
| CH₃ | H | Cl | H | H | N | CH | C=O |
| H | H | Cl | H | H | N | CH | O |
| CH₃ | H | Cl | H | H | N | CH | O |
| CH₃ | H | Cl | H | H | CH | N | C=O |
| CH₃ | H | H | Cl | H | N | CH | C=O |
| CH₃ | H | H | Cl | H | N | CH | O |
| H | H | H | Cl | H | N | CH | O |
| CH₃ | H | H | H | Cl | N | CH | C=O |
| H | H | H | H | Cl | N | CH | O |
| CH₃ | H | H | H | Cl | N | CH | O |
| CH₃ | H | H | H | Cl | CH | N | C=O |
| CH₃ | H | Br | H | H | N | CH | C=O |
| H | H | Br | H | H | N | CH | O |
| CH₃ | H | Br | H | H | N | CH | O |
| CH₃ | H | H | Br | H | N | CH | C=O |
| H | H | H | Br | H | N | CH | O |
| CH₃ | H | H | Br | H | N | CH | O |
| CH₃ | CH₃ | H | H | H | N | CH | C=O |
| H | CH₃ | H | H | H | N | CH | O |
| CH₃ | CH₃ | H | H | H | N | CH | O |
| CH₃ | H | CH₃ | H | H | N | CH | C=O |
| H | H | CH₃ | H | H | N | CH | O |
| CH₃ | H | CH₃ | H | H | N | CH | O |
| CH₃ | H | H | CH₃ | H | N | CH | C=O |
| H | H | H | CH3 | H | N | CH | O |
| CH₃ | H | H | CH3 | H | N | CH | O |
| CH₃ | H | H | CH₃ | H | N⁺-O⁻ | CH | C=O |
| CH₃ | H | H | H | CH3 | N | CH | C=O |
| H | H | H | H | CH3 | N | CH | O |
| CH₃ | H | H | H | CH₃ | N | CH | O |
| CH₃ | H | C₂H₅ | H | H | N | CH | C=O |
| CH₃ | H | C₂H₅ | H | H | N | CH | O |
| H | H | C₂H₅ | H | H | N | CH | O |
| CH₃ | H | -CH(CH₃)₂ | H | H | N | CH | C=O |
| H | H | -CH(CH₃)₂ | H | H | N | CH | O |
| CH₃ | H | -CH(CH₃)₂ | H | H | N | CH | O |
| CH₃ | H | -CH₂CH(CH₃)₂ | H | H | N | CH | C=O |
| H | H | -CH₂CH(CH₃)₂ | H | H | N | CH | O |
| CH₃ | H | -CH₂CH(CH₃)₂ | H | H | N | CH | O |
| CH₃ | H | -C₄H₉-n | H | H | N | CH | C=O |
| CH₃ | H | -C₄H₉-n | H | H | N | CH | O |
| H | H | -C₄H₉-n | H | H | N | CH | O |
| CH₃ | H | -C₄H₉-tert | H | H | N | CH | C=O |
| CH₃ | H | -C₄H₉-tert | H | H | N⁺-O⁻ | CH | C=O |
| H | H | -C₄H₉-tert | H | H | N | CH | O |
| CH₃ | H | -C₄H₉-tert | H | H | N | CH | O |
| CH₃ | H | -C₆H₁₃-n | H | H | N | CH | C=O |
| CH₃ | H | -C₆H₁₃-n | H | H | N | CH | O |
| H | H | -C₆H₁₃-n | H | H | N | CH | O |
| CH₃ | OCH₃ | H | H | H | N | CH | C=O |
| H | OCH₃ | H | H | H | N | CH | O |
| CH₃ | OCH₃ | H | H | H | N | CH | O |
| CH₃ | OCH₃ | H | H | H | CH | N | C=O |
| CH₃ | H | OCH₃ | H | H | N | CH | C=O |
| H | H | OCH₃ | H | H | N | CH | O |
| CH₃ | H | OCH₃ | H | H | N | CH | O |
| CH₃ | H | OCH₃ | H | H | N⁺-O⁻ | CH | C=O |
| CH₃ | H | H | OCH₃ | H | N | CH | C=O |
| H | H | H | OCH₃ | H | N | CH | O |
| CH₃ | H | H | OCH₃ | H | N | CH | O |
| CH₃ | H | H | H | OCH₃ | N | CH | C=O |
| H | H | H | H | OCH₃ | N | CH | O |
| CH₃ | H | H | H | OCH₃ | N | CH | O |
| CH₃ | H | OC₂H₅ | H | H | N | CH | C=O |
| H | H | OC₂H₅ | H | H | N | CH | O |
| CH₃ | H | OC₂H₅ | H | H | N | CH | O |
| CH₃ | H | OC₄H₉n | H | H | N | CH | C=O |
| H | H | OC₄H₉n | H | H | N | CH | O |
| CH₃ | H | OC₄H₉n | H | H | N | CH | O |
| CH₃ | H | Allyl | H | H | N | CH | C=O |
| CH₃ | H | Allyl | H | H | N | CH | O |
| CH₃ | CF₃ | H | H | H | N | CH | C=O |
| H | CF₃ | H | H | H | N | CH | O |
| CH₃ | CF₃ | H | H | H | N | CH | O |
| CH₃ | H | CF₃ | H | H | N | CH | C=O |
| H | H | CF₃ | H | H | N | CH | O |
| CH₃ | H | CF₃ | H | H | N | CH | O |
| CH₃ | H | H | CF₃ | H | N | CH | C=O |
| H | H | H | CF₃ | H | N | CH | O |
| CH₃ | H | H | CF₃ | H | N | CH | O |
| CH₃ | H | H | CF₃ | H | N⁺-O⁻ | CH | C=O |
| CH₃ | H | H | H | CF₃ | N | CH | C=O |
| H | H | H | H | CF₃ | N | CH | O |
| CH₃ | H | H | H | CF₃ | N | CH | O |
| CH₃ | NO₂ | H | H | H | N | CH | C=O |
| H | NO₂ | H | H | H | N | CH | O |
| CH₃ | NO₂ | H | H | H | N | CH | O |
| CH₃ | H | NO₂ | H | H | N | CH | C=O |
| H | H | NO₂ | H | H | N | CH | O |
| CH₃ | H | NO₂ | H | H | N | CH | O |
| CH₃ | H | H | NO₂ | H | N | CH | C=O |
| H | H | H | NO₂ | H | N | CH | O |
| CH₃ | H | H | NO₂ | H | N | CH | O |
| CH₃ | H | C₆H₅ | H | H | N | CH | C=O |
| H | H | C₆H₅ | H | H | N | CH | O |
| CH₃ | H | C₆H₅ | H | H | N | CH | O |
| CH₃ | H | H | C₆H₅ | H | N | CH | C=O |
| H | H | H | C₆H₅ | H | N | CH | O |
| CH₃ | H | H | C₆H₅ | H | N | CH | O |
| CH₃ | H | CONH₂ | H | H | N | CH | C=O |
| CH₃ | H | CONH₂ | H | H | N | CH | O |
| CH₃ | H | CON(CH₃)₂ | H | H | N | CH | C=O |
| CH₃ | H | CON(CH₃)₂ | H | H | N | CH | O |
| CH₃ | F | H | F | H | N | CH | C=O |
| H | F | H | F | H | N | CH | O |
| CH₃ | F | H | F | H | N | CH | O |
| CH₃ | F | H | H | F | N | CH | C=O |
| H | F | H | H | F | N | CH | O |
| CH₃ | F | H | H | F | N | CH | O |
| CH₃ | H | F | F | H | N | CH | C=O |
| H | H | F | F | H | N | CH | O |
| CH₃ | H | F | F | H | N | CH | O |
| CH₃ | H | F | H | F | N | CH | C=O |
| H | H | F | H | F | N | CH | O |
| CH₃ | H | F | H | F | N | CH | O |
| CH₃ | F | H | CH₃ | H | N | CH | C=O |
| H | F | H | CH₃ | H | N | CH | O |
| CH₃ | F | H | CH₃ | H | N | CH | O |
| CH₃ | CH₃ | H | F | H | N | CH | O |
| CH₃ | CH₃ | H | F | H | N | CH | C=O |
| CH₃ | F | H | H | CH₃ | N | CH | C=O |
| CH₃ | F | H | H | CH₃ | N | CH | O |
| CH₃ | CH₃ | H | H | F | N | CH | C=O |
| CH₃ | CH₃ | H | H | F | N | CH | O |
| CH₃ | H | F | H | CH₃ | N | CH | C=O |
| CH₃ | H | F | H | CH₃ | N | CH | O |
| CH₃ | F | H | Cl | H | N | CH | C=O |
| CH₃ | F | H | Cl | H | N | CH | O |
| CH₃ | Cl | H | F | H | N | CH | C=O |
| H | Cl | H | F | H | N | CH | O |
| CH₃ | Cl | H | F | H | N | CH | O |
| CH₃ | H | F | H | Cl | N | CH | C=O |
| CH₃ | H | F | H | Cl | N⁺-O⁻ | CH | C=O |
| CH₃ | H | F | H | Cl | N | CH | O |
| CH₃ | H | Cl | H | F | N | CH | C=O |
| CH₃ | H | Cl | H | F | N | CH | O |
| CH₃ | CH₃O | F | H | H | N | CH | C=O |
| CH₃ | CH₃O | H | F | H | N | CH | C=O |
| CH₃ | CH₃O | H | F | H | N | CH | O |
| CH₃ | F | H | CH₃O | H | N | CH | C=O |
| CH₃ | F | H | CH₃O | H | N | CH | O |
| CH₃ | F | H | H | CH₃O | N | CH | C=O |
| CH₃ | F | H | H | CH₃O | N | CH | O |
| CH₃ | CH₃O | H | H | F | N | CH | C=O |
| CH₃ | CH₃O | H | H | F | N⁺-O⁻ | CH | C=O |
| CH₃ | CH₃O | H | H | F | N | CH | O |
| CH₃ | H | CH₃O | H | F | N | CH | C=O |
| CH₃ | H | CH₃O | F | H | N | CH | C=O |
| CH₃ | Cl | H | Cl | H | N | CH | C=O |
| CH₃ | Cl | H | Cl | H | N | CH | O |
| CH₃ | Cl | H | H | Cl | N | CH | C=O |
| H | Cl | H | H | Cl | N | CH | O |
| CH₃ | Cl | H | H | Cl | N | CH | O |
| CH₃ | H | Cl | Cl | H | N | CH | C=O |
| CH₃ | H | Cl | Cl | H | N | CH | O |
| CH₃ | H | Cl | H | Cl | N | CH | C=O |
| CH₃ | H | Cl | H | Cl | N⁺-O⁻ | CH | C=O |
| CH₃ | H | Cl | H | Cl | CH | N | C=O |
| CH₃ | H | Cl | H | Cl | N | CH | O |
| CH₃ | H | Cl | H | Cl | N⁺-O⁻ | CH | O |
| CH₃ | H | Cl | H | Cl | CH | N | O |
| H | H | Cl | H | Cl | CH | N | O |
| CH₃ | Cl | H | CH₃ | H | N | CH | C=O |
| CH₃ | Cl | H | CH₃ | H | N | CH | O |
| CH₃ | CH₃ | H | Cl | H | N | CH | C=O |
| CH₃ | CH₃ | H | Cl | H | N | CH | O |
| CH₃ | Cl | H | H | CH₃ | N | CH | C=O |
| CH₃ | Cl | H | H | CH₃ | N | CH | O |
| CH₃ | CH₃ | H | H | Cl | N | CH | C=O |
| CH₃ | CH₃ | H | H | Cl | N | CH | O |
| CH₃ | H | Cl | CH₃ | H | N | CH | C=O |
| CH₃ | H | Cl | CH₃ | H | N | CH | O |
| CH₃ | H | Cl | H | CH₃ | N | CH | C=O |
| CH₃ | H | Cl | H | CH₃ | N | CH | O |
| CH₃ | H | CH₃ | H | Cl | N | CH | C=O |
| CH₃ | H | CH₃ | H | Cl | N | CH | O |
| CH₃ | Cl | H | CH₃O | H | N | CH | C=O |
| H | Cl | H | CH₃O | H | N | CH | O |
| CH₃ | Cl | H | CH₃O | H | N | CH | O |
| CH₃ | CH₃O | H | Cl | H | N | CH | C=O |
| CH₃ | CH₃O | H | Cl | H | N | CH | O |
| CH₃ | Cl | H | H | CH₃O | N | CH | C=O |
| H | Cl | H | H | CH₃O | N | CH | O |
| CH₃ | Cl | H | H | CH₃O | N | CH | O |
| CH₃ | H | Cl | CH₃O | H | N | CH | C=O |
| CH₃ | H | Cl | CH₃O | H | N | CH | O |
| CH₃ | H | CH₃O | Cl | H | N | CH | C=O |
| CH₃ | H | CH₃O | Cl | H | N | CH | O |
| CH₃ | H | Cl | H | CH₃O | N | CH | C=O |
| CH₃ | H | Cl | H | CH₃O | N | CH | O |
| CH₃ | H | CH₃O | H | Cl | N | CH | C=O |
| CH₃ | H | CH₃O | H | Cl | N | CH | O |
| CH₃ | Cl | H | C₂H₅O | H | N | CH | C=O |
| CH₃ | Cl | H | C₂H₅O | H | N⁺-O⁻ | CH | C=O |
| CH₃ | CH₃ | H | CH₃ | H | N | CH | C=O |
| H | CH₃ | H | CH₃ | H | N | CH | O |
| CH₃ | CH₃ | H | CH₃ | H | N | CH | O |
| CH₃ | H | CH₃ | CH₃ | H | N | CH | C=O |
| CH₃ | H | CH₃ | CH₃ | H | N | CH | O |
| CH₃ | H | CH₃ | H | CH₃ | N | CH | C=O |
| CH₃ | H | CH₃ | H | CH₃ | N | CH | O |
| CH₃ | CH₃ | H | H | CH₃ | N | CH | C=O |
| CH₃ | CH₃ | H | H | CH₃ | N | CH | O |
| CH₃ | CH₃ | H | H | CH₃ | CH | N | C=O |
| CH₃ | CH₃O | CH₃O | H | H | N | CH | C=O |
| H | CH₃O | CH₃O | H | H | N | CH | O |
| CH₃ | CH₃O | CH₃O | H | H | N | CH | O |
| CH₃ | CH₃O | H | CH₃O | H | N | CH | C=O |
| CH₃ | CH₃O | H | CH₃O | H | N | CH | O |
| CH₃ | H | CH₃O | CH₃O | H | N | CH | C=O |
| CH₃ | H | CH₃O | CH₃O | H | N | CH | O |
| CH₃ | CH₃O | H | H | CH₃O | N | CH | C=O |
| H | CH₃O | H | H | CH₃O | N | CH | O |
| CH₃ | CH₃O | H | H | CH₃O | N | CH | O |
| CH₃ | H | CH₃O | H | CH₃O | N | CH | C=O |
| CH₃ | H | CH₃O | H | CH₃O | N⁺-O⁻ | CH | C=O |
| CH₃ | H | CH₃O | H | CH₃O | CH | N | C=O |
| CH₃ | H | CH₃O | H | CH₃O | CH | N⁺-O⁻ | C=O |
| H | H | CH₃O | H | CH₃O | N | CH | O |
| CH₃ | H | CH₃O | H | CH₃O | N | CH | O |

Die Verbindungen I bzw. I' und die sie enthaltenden herbiziden Mittel sowie deren umweltverträgliche Salze von Alkalimetallen und Erdalkalimetallen können in Kulturen wie Weizen, Reis, Mais, Soja und Baumwolle Unkräuter und Schadgräser sehr gut bekämpfen, ohne die Kulturpflanzen zu schädigen, ein Effekt, der vor allem auch bei niedrigen Aufwandmengen auftritt.

Die Verbindungen I bzw. I' und die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I bzw. I' eignen sich allgemein zur Herstellung von direkt versprühbaren Lösungen, Emulsionen, Pasten oder Öldis persionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, alkylierte Benzole oder deren Derivate, Methanol, Ethanol, Propanol, Butanol, Cyclohexanol, Cyclohexanon, Chlorbenzol oder stark polare Lösungsmittel, wie N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctyl-phenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen alkylether Laurylalkoholpolyglykoletheracetat, Sorbitester, Lignin-Sulfitäblaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten im allgemeinen zwischen 0,01 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR/HPLC/GC-Spektrum) eingesetzt.

Beispiele für solche Zubereitungen sind:
I. 20 Gew.-Teile der Verbindung Nr. 1 werden in einer Mischung gelöst, die aus 80 Gew.-Teilen alkyliertem Benzol, 10 Gew.-Teilen des Anlagerungsproduktes von 8 bi 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes von 40 Mol Ethylenoxid an 1 Mol Ricinusöl besteht. Durch Ausgießen und feines Verteilen der Lösung in 100 000 Gew.-Teilen Wasser erhält man eine wäßrige Dispersion, die 0,02 Gew.-% des Wirkstoffs enthält.
II. 20 Gew.-Teile der Verbindung Nr. 1 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.
III. 20 Gew.-Teile des Wirkstoffs Nr. 1 werden in einer Mischung gelöst, die aus 25 Gew.-Teilen Cyclohexanon, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinus besteht. Durch Eingießen und feines Verteilen der Lösung in 100 000 Gew.-Teilen Wasser erhält man eine wäßrige Dispersion, die 0,02 Gew.-% des Wirkstoffs enthält.
IV. 20 Gew.-Teile des Wirkstoffs Nr. 1 werden mit 3 Gew.-Teilen des Natriumsalzes der Diisobutylnaphthalin-α-sulfonsäure, 17 Gew.-Teile des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser enthält man eine Spritzbrühe, die 0,1 Gew.-% des Wirkstoffs enthält.
V. 3 Gew.-Teile des Wirkstoffs Nr. 1 werden mit 97 Gew.-Teilen feinteiligem Kaolin vermischt. Man erhält auf diese Weise ein Stäubemittel, das 3 Gew.-% des Wirkstoffs enthält.
VI. 20 Gew.-Teile des Wirkstoffs Nr. 1 werden mit 2 Gew.-Teilen Calciumsalz der Dodecylbenzolsulfonsäure, 8 Gew.-Teilen Fettalkohol-polyglykolether, 2 Gew.-Teilen Natriumsalz eines Phenol-Harnstoff-Formaldehyd-Kondensates und 68 Gew.-Teilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 5,0, vorzugsweise 0,01 bis 2,0 kg/ha aktive Substanz (a.S.).

In Anbetracht der Vielseitigkeit der Applikationsmethoden können die erfindungsgemäßen Verbindungen I und I' bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica),

Cucumis sativus, Cynodon dactylon, Daucus carota, Elaeis guineensis, Fragaria vesca, Glycine max, Gossypium hirsutum (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N. rustica), Olea europaea,Oryza sativa,Phaseolus lunatus, Phaseolus mungo, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Die wachstumsregulierend wirksamen Verbindungen I bzw. I' können praktisch alle Entwicklungsstadien einer Pflanze verschiedenartig beeinflussen. Die Wirkungsvielfalt der Pflanzenwachstumsregulatoren hängt ab vor allem
a) von der Pflanzenart und -sorte,
b) vom Zeitpunkt der Applikation, bezogen auf das Entwicklungsstadium der Pflanze und von der Jahreszeit,
c) von dem Applikationsort und -verfahren (z.B. Samenbeize, Bodenbehandlung, Blattapplikation oder Stamminjektion bei Bäumen),
d) von klimatischen Faktoren, z.B. Temperatur, Niederschlagsmenge, außerdem auch Tageslänge und Lichtintensität,
e) von der Bodenbeschaffenheit (einschließlich Düngung),
f) von der Formulierung bzw. Anwendungsform des Wirkstoffs und schließlich
g) von der angewendeten Konzentration der aktiven Substanz.

Aus der Reihe der verschiedenartigen Anwendungsmöglichkeiten der Pflanzenwachstumsregulatoren der Formel I bzw. I' im Pflanzenanbau, in der Landwirtschaft und im Gartenbau, werden einige nachstehend erwähnt.
A. Mit den erfindungsgemäß verwendbaren Verbindungen läßt sich das vegetative Wachstum der Pflanzen stark hemmen, was sich insbesondere in einer Reduzierung des Längenwachstums äußert.
   Die behandelten Pflanzen weisen demgemäß einen gedrungenen Wuchs aus; außerdem ist eine dunklere Blattfärbung zu beobachten.
   Als vorteilhaft für die Praxis erweist sich eine verminderte Intensität des Wachstums von Gräsern sowie lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.
   Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.
   Bei Obst- und anderen Bäumen lassen sich mit den Wachstumsregulatoren Schnittkosten einsparen. Außerdem kann die Alternanz von Obstbäumen durch Wachstumsregulatoren gebrochen werden.
   Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.
   Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generative Phase neigen. Auch bei anderen Kulturen, z.B. Wintergetreide, ist es vorteilhaft, wenn die Bestände durch Behandlung mit den erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt bzw. Pflanzenmasse - dem Befall mit verschiedenen Krankheiten (z.B. Pilzkrankheit) vorgebeugt werden.
B. Mit den Wachstumsregulatoren lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Citrusfrüchten zu erhöhen, den Proteingehalt in Getreide oder Soja zu steigern oder Gummibäume zum vermehrten Latexfluß zu stimulieren.
   Dabei können die Verbindungen der Formel I bzw. I' Ertragssteigerungen durch Eingriffe in den pflanzlichen Stoffwechsel bzw. durch Förderung oder Hemmung des vegetativen und/oder des generativen Wachstums verursachen.
C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.
   Von wirtschaftlichem Interesse ist beispielsweise die Ernteerleichterung, die durch das zeitlich konzentrierte Abfallen oder Vermindern der Haftfestigkeit am Baum bei Citrusfrüchten, Oliven oder bei anderen Arten und Sorten von Kern-, Stein- und Schalenobst ermöglicht wird. Derselbe Mechanismus, d.h. die Förderung der Ausbildung von Trenngewebe zwischen Frucht-, bzw. Blatt- und Sproßteil der Pflanze ist auch für ein gut kontrollierbares Entblättern von Nutzpflanzen wie beispielsweise Baumwolle wesentlich.
D. Mit Wachstumsregulatoren kann weiterhin der Wasserverbrauch von Pflanzen reduziert werden. Durch den Einsatz der erfindungsgemäßen Substanzen läßt sich die Intensität der Bewässerung reduzieren und damit eine kostengünstigere Bewirtschaftung durchführen, weil u.a. die Öffnungsweite der Stomata reduziert wird, eine dickere Epidermis und Cuticula ausgebildet werden, die Durchwurzelung des Bodens verbessert wird und das Mikroklima im Pflanzenbestand durch einen kompakteren Wuchs günstig beeinflußt wird.

Besonders gut eignen sich sich Verbindungen I bzw. I' zur Halmverkürzung von Kulturpflanzen wie Gerste, Raps und Weizen.

Die erfindungsgemäß zu verwendenden Wirkstoffe der Formel I bzw. I' können den Kulturpflanzen sowohl vom Samen her (als Saatgutbeizmittel) als auch über den Boden, d.h. durch die Wurzel sowie - besonders bevorzugt - durch Spritzung über das Blatt zugeführt werden.

Die Aufwandmenge an Wirkstoff ist infolge der hohen Pflanzenverträglichkeit nicht kritisch. Die optimale Aufwandmenge variiert je nach Bekämpfungsziel, Jahreszeit, Zielpflanzen und Wachstumsstadien.

Bei der Saatgutbehandlung werden im allgemeinen Wirksboffmengen von 0.001 bis 50 g, vorzugsweise 0.01 bis 10 g, je Kilogramm Saatgut benötigt.

Für die Blatt- und Bodenbehandlung sind im allgemeinen Gaben von 0.001 bis 10 kg/ha, bevorzugt 0.01 bis 3 kg/ha, insbesondere 0.01 bis 0.5 kg/ha als ausreichend zu betrachten.

Zur Verbreiterung des Wirkungsspektrums und zur Erzielung synergistischer Effekte können die Azaanthrachinone und Azaxanthone der allgemeinen Formel I bzw. I' mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazolinone, Sulfonamide, Sulfonylharnstoffe, Aryloxy-, Heteroaryloxyphenoxypropionsäuren sowie deren Salze, Ester und Amide und andere in Betracht.

Außerdem kann es von Nutzen sein, die Verbindungen I bzw. I' allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

Die Herstellung und die Verwendung der Wirkstoffe I bzw. I' geht aus den nachfolgenden Beispielen hervor.

### Herstellungsbeispiele:

### Beispiel 1

### 4-Methylbenzo[g]chinolin-5,10-dion (Cleistopholin)

Einer Lösung von 30 g 2-Brom-1,4-naphthochinon (0,126 mol) in 250ml Xylol wurden 18,5 g (0,165 mol) Crotonaldehyd-dimethylhydrazon gelöst in 50 ml Xylol zugegeben. Nach 8-stündigem Rühren bei 140°C wurde das Gemisch auf 20°C abgekühlt und mit 2 N H₂SO₄ extrahiert. Die vereinigten, wäßrigen Extrakte wurden mit festem Kaliumcarbonat auf pH 9 gebracht und mit Ethylacetat extrahiert. Die vereinigten organischen Phasen wurden über K₂CO₃ getrocknet und im Vakuum eingeengt. Man erhielt 13,4 g (36,3% d. Theorie) 4-Methylbenzo[g]chinolin-5,10-dion als schwach gelbe Kristalle vom Schmp. 204-206°C.

### Beispiel 2

### 7-Methyl-5H-[1]benzopyrano[2,3-b]pyridin-5-on

Die Mischung von 49,5 g (0,19 mol) 2-(4-Methylphenoxy)-nicotinsäure-ethylester und 460 g Polyphosphorsäure wurden 8 Stunden bei 140°C gerührt, abgekühlt, in 800 g Eis eingerührt, mit einer 28%igen, wäßrigen Ammoniaklösung alkalisch gestellt und der ausgefallene Niederschlag abgesaugt und nacheinander mit Wasser und mit wenig kaltem Ethanol gewaschen und getrocknet. Man erhielt 29,3 g (73 % d. Theorie) 7-Methyl-5H[1]benzopyrano[2,3-b]pyridin-5-on als gelbliche Kristalle vom Schmp. 155-157°C.

Analog zu den Herstellungsbeispielen 1 und 2 und gemäß den allgemeinen Angaben zur Herstellung erhielt man die in Tabelle 2 aufgeführten Verbindungen der Formel I bzw. I'.

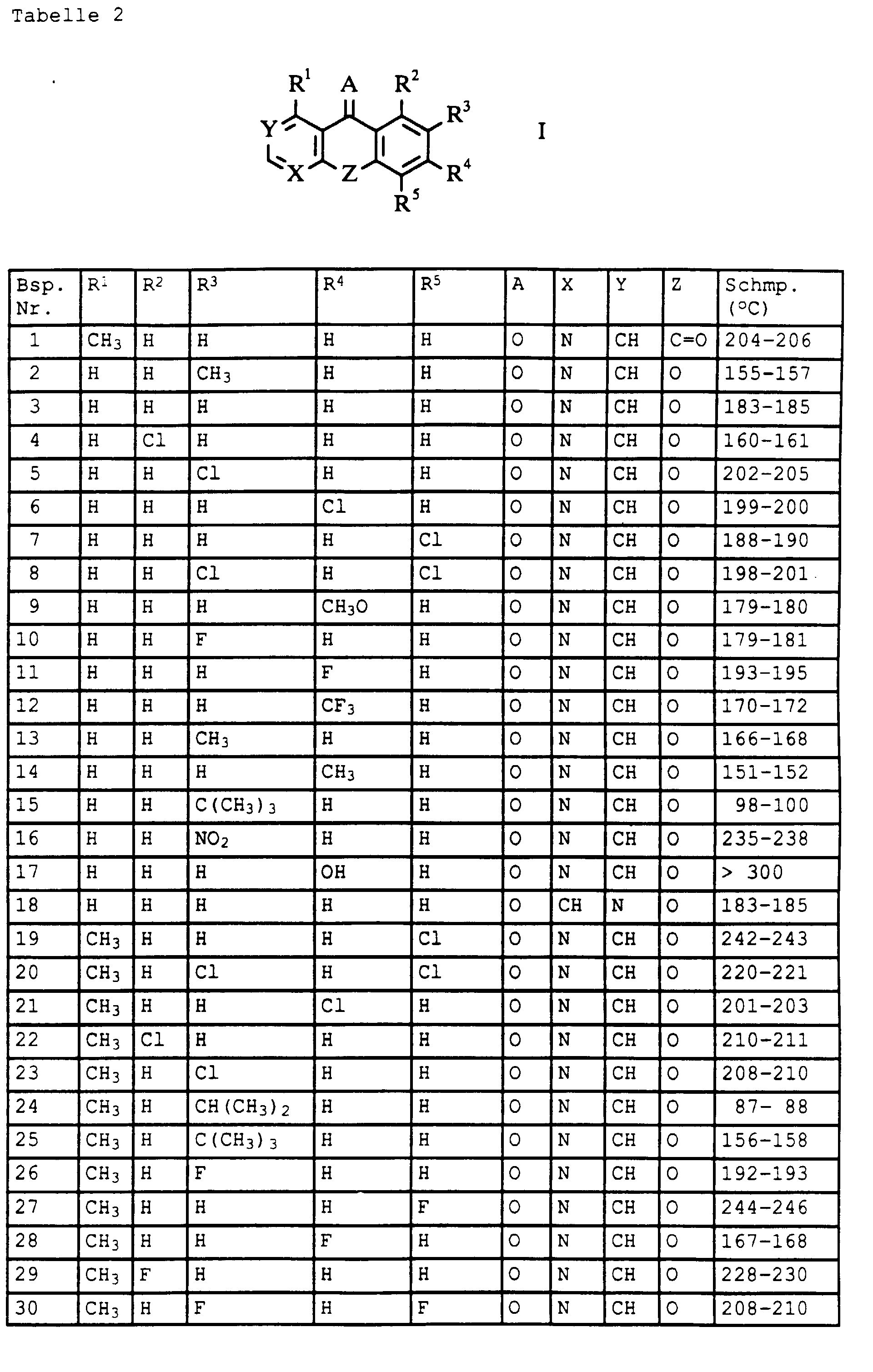

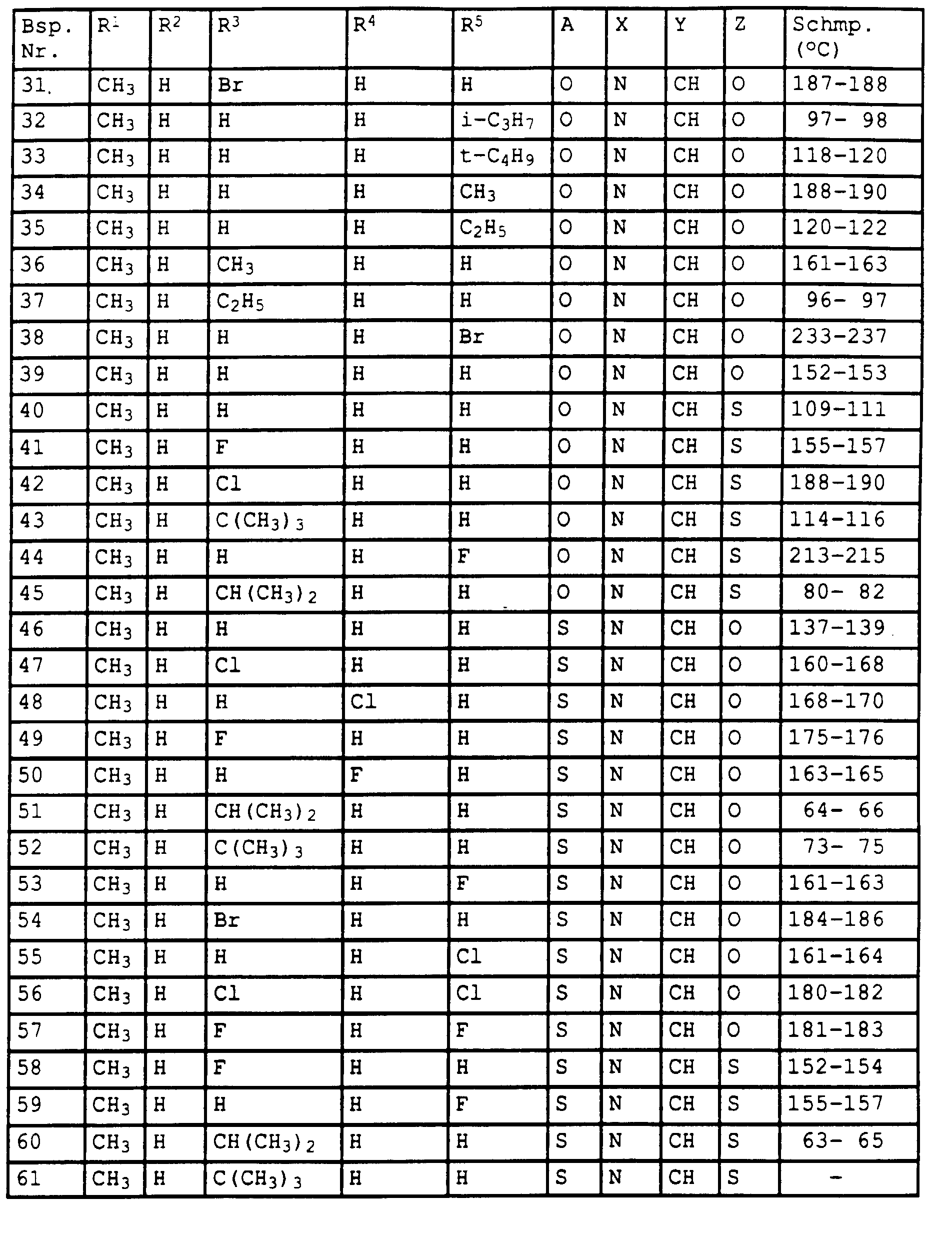

### Anwendungsbeispiele

Die herbizide Wirkung der Azaanthrachinone und Azaxanthone der Formel I bzw. I' ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt. Die Aufwandmenge für die Nachauflaufbehandlung betrug 3.0 kg/ha a.S.

Die Pflanzen wurden artenspezifisch bei Temperaturen von 10-25°C bzw. 20-35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| | | |
|---|---|---|
| Lateinischer Name | Deutscher Name | Englischer Name |
| Centaurea cyanus | Kornblume | cornflower |
| Echinochloa crus-galli | Hühnerhirse | barnyardgrass |
| Ipomoea ssp. | Prunkwinde | morningglory |

Die Ergebnisse belegen die gute herbizide Wirkung des Cleistopholins. Eine sehr gute wachstumshemmende Wirkung wird von dem unter Beispiel 7 aufgeführten Azaxanthon in den Kulturpflanzen Winterweizen und Raps erzielt.

## Patentansprüche

1. Neue Azaanthrachinone und Azaxanthone der Formel I 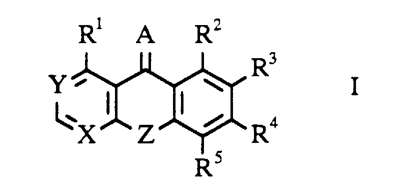 in der X, Y, Z und die Reste R¹ bis R⁵ folgende Bedeutung haben:
R¹ Wasserstoff, Methyl,
R²-R⁵
a) Wasserstoff,
b) Halogen,
c) Nitro,
d) CONH₂ und CONR⁶R⁷, wobei R⁶und R⁷ C₁-C₄-Alkyl bedeuten,
e) C₁-C₈-Alkyl, das ein bis drei der folgenden Substituenten tragen kann: Halogen, Hydroxy, C₁-C₆-Alkoxy,
f) C₃-C₆-Alkenyl,
g) C₁-C₄-Alkoxy,
h) Hydroxy,
i) Amino oder NR⁶R⁷,
j) Phenyl, das ein bis fünf Halogenatome oder ein bis drei der folgenden Substituenten tragen kann: Nitro, C₁-C₄-Alkoxy, C₁-C₄-Alkyl oder C₁-C₄-Halogenalkyl;
X, Y N, N⁺-O⁻ oder CH, mit der Maßgabe, daß ausschließlich ein Stickstoffatom bzw. eine N-oxidgruppe im Ring enthaltend ist;
Z, A Sauerstoff cder Schwefel;
sowie die Salze von I mit solchen Säuren, welche die herbizide bzw. die pflanzenwachstumsregulierende Wirkung von I nicht beeinträchtigen; mit der Maßgabe, daß die Azaxanthone der Formel Ia 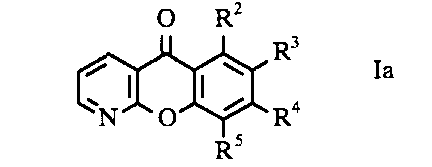 in der R², R³, R⁴, R⁵ gleich oder verschieden sind und unabhängig voneinander Wasserstoff, Fluor, Chlor, Methyl, Methoxy, CF₃, CCl₃, tert.-C₄H₉, NO₂, NH₂ und Phenyl bedeuten, vom Anspruch ausgeschlossen sind.

2. Verbindungen der Formel I gemäß Anspruch 1, in der R¹ Methyl bedeutet.

3. Verbindungen der Formel I gemäß Anspruch 1, in der X Stickstoff oder eine N-oxidgruppe bedeutet und Y für CH steht.

4. Herbizides Mittel, enthaltend eine herbizid wirksame Menge einer Verbindung der Formel I gemäß Anspruch 1.

5. Mittel zur Regulierung des Pflanzenwachstums, enthaltend eine bioregulativ wirksame Menge einer Verbindung der Formel I, gemäß Anspruch 1 und inerte Zusatzstoffe.

6. Verfahren zur Bekämpfung unerwünschten Pflanzenwuchses, dadurch gekennzeichnet, daß man eine herbizid wirksame Menge einer Verbindung der Formel I' 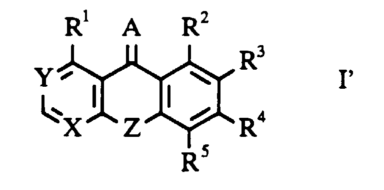 in der X, Y, Z, A und die Reste R¹ bis R⁵ folgende Bedeutung haben:
R¹ Wasserstoff, Methyl,
R²-R⁵
a) Wasserstoff,
b) Halogen,
c) Nitro,
d) COOR⁶, wobei R⁶ für C₁-C₄-Alkyl steht,
e) CONH₂ und CONR⁶R⁷, wobei R⁶ und R⁷ C₁-C₄-Alkyl bedeuten,
f) C₁-C₈-Alkyl, das ein bis drei der folgenden Substituenten tragen kann: Halogen, Hydroxy, C₁-C₆-Alkoxy,
g) C₃-C₆-Alkenyl,
h) C₁-C₄-Alkoxy,
i) Hydroxy,
j) Amino oder NR⁶R⁷,
k) Phenyl, das ein bis fünf Halogenatome oder ein bis drei der folgenden Substituenten tragen kann: Nitro, C₁-C₄-Alkoxy, C₁-C₄-Alkyl oder C₁-C₄-Halogenalkyl;
X, Y N, N⁺-O⁻ oder CH, mit der Maßgabe, daß ausschließlich ein Stickstoffatom bzw. eine N-oxidgruppe im Ring enthaltend ist;
Z Sauerstoff, Schwefel oder eine Gruppe C=O;
A Sauerstoff oder schwefel;
sowie die Salze von I mit solchen Säuren, welche die herbizide bzw. die pflanzenwachstumsregulierende Wirkung von I nicht beeinträchtigen, auf Pflanzen, deren Lebensraum oder auf Saatgut einwirken läßt.

7. Verfahren zur Regulierung des Pflanzenwachstums, dadurch gekennzeichnet, daß man die Pflanzen, ihren Lebensraum und/oder ihre Samen mit einer bioregulatorisch wirksamen Menge einer Verbindung der Formel I' gemäß Anspruch 6 behandelt.

8. Verfahren zur Bekämpfung unerwünschten Pflanzenwuchses und zur Regulierung des Pflanzenwachstums, dadurch gekennzeichnet, daß man die Pflanzen, ihren Lebensraum und/oder ihre Samen mit einer bioregulatorisch wirksamen Menge an Cleistopholin behandelt.

9. Verwendung von Verbindungen der Formel I' gemäß Anspruch 6, in der R¹ Wasserstoff, R² Wasserstoff oder Halogen, R³ Wasserstoff, Halogen, C₁-C₄-Alkyl oder Nitro, R⁴ Wasserstoff, Halogen, C₁-C₄-Alkyl oder -Alkoxy oder Hydroxy, R⁵ Wasserstoff oder Halogen, X N, Y CH und Z Sauerstoff, Schwefel oder C=O und A Sauerstoff oder Schwefel bedeuten, zur Herstellung von Mitteln zur Bekämpfung unerwünschten Pflanzenwuchses oder zur Regulierung des Pflanzenwuchses.

10. Verwendung von Cleistopholin zur Herstellung von Mitteln zur Bekämpfung unerwünschten Pflanzenwuchses oder zur Regulierung des Pflanzenwuchses.

## Claims

1. A novel azaanthraquinone or azaxanthone of the formula I 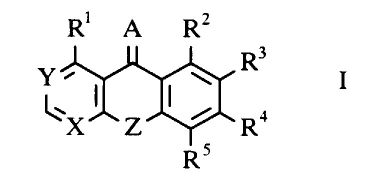 where
R¹ is hydrogen or methyl,
R²-R⁵ are each
a) hydrogen,
b) halogen,
c) nitro,
d) CONH₂ or CONR⁶R⁷, where R⁶ and R⁷ are each C₁-C₄-alkyl,
e) C₁-C₈-alkyl which may carry from one to three of the following substituents: halogen, hydroxyl and C₁-C₆-alkoxy,
f) C₃-C₆-alkenyl,
g) C₁-C₄-alkoxy,
h) hydroxyl,
i) amino or NR⁶R⁷, or
j) phenyl which may carry from one to five halogen atoms or from one to three of the following substituents: nitro, C₁-C₄-alkoxy, C₁-C₄-alkyl or C₁-C₄-haloalkyl;
X and Y are each N, N⁺-O⁻ or CH, with the proviso that only one nitrogen atom or one N-oxide group is present in the ring, and
Z and A are each oxygen or sulfur,
and the salts of I with those acids which do not adversely affect the herbicidal or plant growth-regulating action of I, with the proviso that the azaxanthones of the formula Ia 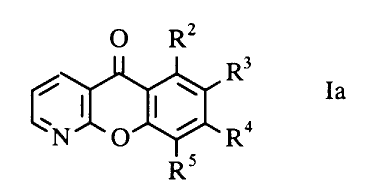 where R², R³, R⁴ and R⁵ are identical or different and, independently of one another, are each hydrogen, fluorine, chlorine, methyl, methoxy, CF₃, CCl₃, tert-C₄H₉, NO₂, NH₂ or phenyl, are excluded from the claim.

2. A compound of the formula I as claimed in claim 1, in which R¹ is methyl.

3. A compound of the formula I as claimed in claim 1, in which X is nitrogen or an N-oxide group and Y is CH.

4. A herbicide containing a herbicidal amount of a compound of the formula I as claimed in claim 1.

5. A plant growth regulator containing a bioregulatory amount of a compound of the formula I as claimed in claim 1 and inert additives.

6. A method for controlling undesirable plant growth, wherein a herbicidal amount of a compound of the formula I' 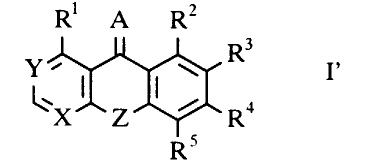 where
R¹ is hydrogen or methyl,
R²-R⁵ are each
a) hydrogen,
b) halogen,
c) nitro,
d) COOR⁶, where R⁶ is C₁-C₄-alkyl,
e) CONH₂ or CONR⁶R⁷, where R⁶ and R⁷ are each C₁-C₄-alkyl,
f) C₁-C₈-alkyl which may carry from one to three of the following substituents: halogen, hydroxyl and C₁-C₆-alkoxy,
g) C₃-C₆-alkenyl,
h) C₁-C₄-alkoxy,
i) hydroxyl,
j) amino or NR⁶R⁷, or
k) phenyl which may carry from one to five halogen atoms or from one to three of the following substituents: nitro, C₁-C₄-alkoxy, C₁-C₄-alkyl or C₁-C₄-haloalkyl;
X and Y are each N, N⁺-O⁻ or CH, with the proviso that only one nitrogen atom or one N-oxide group is present in the ring;
Z is oxygen, sulfur or a C=O group and
A is oxygen or sulfur;
and the salts of I with those acids which do not adversely affect the herbicidal or plant growth-regulating action of I, is allowed to act on plants or their habitat or on seed.

7. A method for regulating plant growth, wherein the plants, their habitat and/or their seeds is or are treated with a bioregulatory amount of a compound of the formula I' as claimed in claim 6.

8. A method for controlling undesirable plant growth and for regulating plant growth, wherein the plants, their habitat and/or their seeds is or are treated with a bioregulatory amount of cleistopholine.

9. Use of a compound of the formula I' as claimed in claim 6, in which R¹ is hydrogen, R² is hydrogen or halogen, R³ is hydrogen, halogen, C₁-C₄-alkyl or nitro, R⁴ is hydrogen, halogen, C₁-C₄-alkyl, C₁-C₄-alkoxy or hydroxyl, R⁵ is hydrogen or halogen, X is N, Y is CH, Z is oxygen, sulfur or C=O and A is oxygen or sulfur, for the preparation of compositions for controlling undesirable plant growth or for regulating plant growth.

10. Use of cleistopholine for the preparation of compositions for controlling undesirable plant growth or for regulating plant growth.

## Revendications

1. Nouvelles azaanthraquinones et azaxanthones de formule I 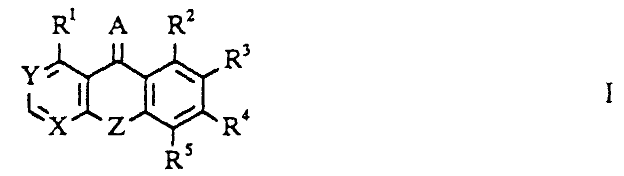 dans laquelle X, Y, Z et R¹ à R⁵ ont les significations suivantes :
R¹ l'hydrogène, un groupe méthyle,
R² à R⁵
a) l'hydrogène,
b) un halogène,
c) un groupe nitro,
d) un groupe CONH₂ ou CONR⁶R⁷, R⁶ et R⁷ représentant des groupes alkyle en C1-C4,
e) un groupe alkyle en C1-C8 qui peut porter un à trois des substituants suivants : halogènes, hydroxy, alcoxy en C1-C6,
f) un groupe alcényle en C3-C6,
g) un groupe alcoxy en C1-C4,
h) un groupe hydroxy,
i) un groupe amino ou NR⁶R⁷,
j) un groupe phényle qui peut porter un à cinq atomes d'halogènes ou un à trois des substituants suivants ; nitro, alcoxy en C1-C4, alkyle en C1-C4 ou halogénoalkyle en C1- C4;
X, Y : N, N⁺-O⁻ ou CH, sous réserve que le cycle ne contient qu'un atome d'azote ou qu'un groupe N-oxyde ;
Z, A : l'oxygène ou le soufre;
ainsi que les sels des composés I et d'acides qui n'affectent pas les propriétés herbicides et régulatrices de croissance des végétaux des composés I; sous réserve que les azaxanthones de formule la 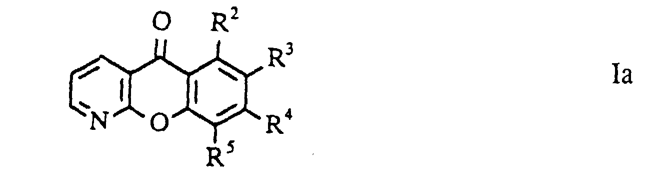 dans laquelle R², R³, R⁴, R⁵, ayant des significations identiques ou différentes, représentent chacun, indépendamment les uns des autres, l'hydrogène, le fluor, le chlore, un groupe méthyle, méthoxy, CF₃, CCl₃, tert-C₄H₉, NO₂, NH₂ ou phényle, sont exclus de la revendication.

2. Composés de formule I selon la revendication 1, dans laquelle R¹ représente un groupe méthyle.

3. Composés de formule I selon la revendication 1, dans laquelle X représente l'azote ou un groupe N-oxyde et Y représente CH.

4. Produit herbicide contenant une quantité herbicide efficace d'un composé de formule I selon la revendication 1.

5. Produit pour la régulation de la croissance des végétaux, contenant une quantité biorégulatrice efficace d'un composé de formule I selon la revendication 1 et des additifs inertes.

6. Procédé pour combattre les croissances de végétaux indésirables, caractérisé par le fait que l'on fait agir sur les végétaux, leur habitat ou leurs semences, une quantité herbicide efficace d'un composé de formule I' 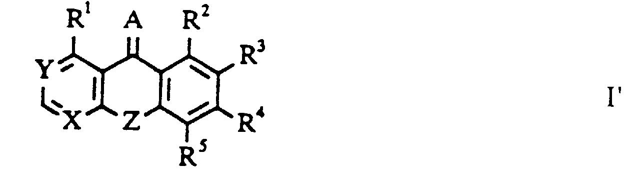 dans laquelle X, Y, Z, A et R¹ à R⁵ ont les significations suivantes :
R¹ : l'hydrogène, un groupe méthyle,
R² à R⁵ :
a) l'hydrogène
b) un halogène,
c) un groupe nitro,
d) un groupe COOR⁶ dans lequel R⁶ représente un groupe alkyle en C1-C4,
e) un groupe CONH₂ ou CONR⁶R⁷, R⁶ et R⁷ représentant des groupes alkyle en C1-C4,
f) un groupe alkyle en C1-C8 qui peut porter un à trois des substituants suivants : halogènes, hydroxy, alcoy en C1-C6,
g) un groupe alcényle en C3-C6,
h) un groupe alcoxy en C1-C4,
i) un groupe hydroxy,
j) un groupe amino ou NR⁶R⁷,
k) un groupe phényle qui peut porter un à cinq atomes d'halogènes ou un à trois des substituants suivants : nitro, alcoxy en C1-C4, alkyle en C1-C4 ou halogénoalkyle en C1-C4;
X, Y : N, N⁺-O⁻ ou CH sous réserve que le cycle ne contient qu'un atome d'azote ou qu'un groupe N-oxyde;
Z : l'oxygène, le soufre ou un groupe C=O;
A : l'oxygène ou le soufre;
ou d'un sel d'un composé I et d'acide qui n'affecte pas les propriétés herbicides et régulatrices de la croissance des végétaux des composés I.

7. Procédé pour la régulation de la croissance des végétaux, caractérisé par le fait que l'on traite les végétaux, leur habitat et/ou leurs semences par une quantité biorégulatrice efficace d'un composé de formule I' selon la revendication 6.

8. Procédé pour combattre les croissances de végétaux indésirables et pour la régulation de la croissance des végétaux, caractérisé par le fait que l'on traite les végétaux, leur habitat et/ou leurs semences par une quantité biorégulatrice efficace de cléistopholine.

9. Utilisation des composés de formule I' selon la revendication 6, dans laquelle R¹ représente l'hydrogène, R² l'hydrogène ou un halogène, R³ l'hydrogène, un halogène, un groupe alkyle en C1-C4 ou nitro, R⁴ l'hydrogène, un halogène, un groupe alkyle ou alcoxy en C1-C4 ou un groupe hydroxy, R⁵ l'hydrogène ou un halogène, X représente N, Y représente CH et Z l'oxygène, le soufre ou un groupe C=O et A l'oxygène ou le soufre, pour la préparation de produits servant à la lutte contre les croissances de végétaux indésirables ou à la régulation de la croissance des végétaux.

10. Utilisation de cléistopholine pour la préparation de produits de lutte contre la croissance des végétaux parasites ou pour la régulation de la croissance des végétaux.
